# EUROPEAN PATENT APPLICATION

(11) **EP 4 571 647 A1**
(43) Date of publication of application: **18.06.2025**
(21) Application number: 24219765.5
(22) Date of filing: 13.12.2024
(51) Int. Cl.: G06T 7/50, A61C 9/00, H04N 19/00

(54) **GENERATING A HIGH-RESOLUTION SCAN OF AN INTRAORAL CAVITY**

(30) Priority: 13.12.2023 US 202318538035
(71) Applicant: Dentsply Sirona Inc., York, PA 17401-2991 (US)
(72) Inventor: SCHNABEL, Ruwen, 64625 Bensheim (DE); ADAMSON, Anders, 64625 Bensheim (DE)
(74) Representative: Aldridge, Henry Alexander

(57) **Abstract**

An aspect includes generating a high-resolution surface scan of an intraoral cavity, including sampling a surface of the intraoral cavity by capturing a number of single images of the surface with a scanner, detecting a bandwidth of a transmission, reducing a resolution of one or more single images on detecting that the bandwidth is lower than a threshold, by converting the single images into corresponding low-resolution frames and residual frames, transmitting the corresponding low-resolution frames, sequentially stitching, at a first time period, the low-resolution frames to continue the surface scan to generate a stitched model, and selectively replacing, at a second time period, low-resolution frames with the corresponding residual frames within the stitched model. A high-resolution surface scan of the intraoral cavity is then generated.

## Description

### BACKGROUND

### Technical Field

The present disclosure generally relates to intraoral scanning and, more particularly to generating a high-resolution scan of an intraoral cavity under variable bandwidth conditions.

### Description of the Related Art

In the realm of intraoral imaging in a wireless setting, the use of intraoral scanners for digital impressions faces challenges when the transmission bandwidth intermittently drops below the rate at which data is being acquired, posing a hindrance to seamless data transfer. The conventional approach resorts to dropping captured frames to adapt the amount of data in the transmission bandwidth. However, this may introduce disruptions in the scan-flow, compelling the operator to intervene by repositioning the scanner and, in some cases, re-acquiring specific segments of the intraoral cavity. These interruptions may not only impede the efficiency of the scanning process but may also result in a less-than-optimal user experience for users and patients alike.

### SUMMARY

According to an embodiment of the present disclosure, a method is disclosed that includes sampling a surface of the intraoral cavity by capturing a plurality of single images of the surface with a scanner, detecting a bandwidth of a transmission, reducing a resolution of one or more single images of the plurality of single images associated with at least one region of the surface on detecting that the bandwidth is lower than a predetermined threshold, by converting the one or more single images into respective low-resolution frames and residual frames associated with the at least one region, and sequentially transmitting the respective low-resolution frames to a receiver at the bandwidth lower than the predetermined threshold. Further, the method includes sequentially stitching, by the receiver at a first time period, the respective low-resolution frame to continue the surface scan to generate a stitched model, selectively updating, at a second time period subsequent to the first time period, the respective low-resolution frames based corresponding residual frames within the stitched model and generating the high-resolution surface scan of the intraoral cavity. The updating may include combining the residual frames with the low resolution frames to arrive back at the original high resolution frame. Alternatively, if the original high resolution frames are kept, the low resolution frames can be replaced.

In one embodiment, the method may also include registering and stitching the respective low-resolution frames associated with the at least one region in a sequence with the plurality of the single images of other regions to generate the stitched model of the surface.

In one embodiment, the method may also include sequentially transmitting the respective low-resolution frames with associated IDs, storing the corresponding residual frames of the one or more single images within an image buffer of the scanner, identify the at least one region with a sampling rate lower than a predetermined sampling rate, and request to receive the corresponding residual frames of the respective low-resolution frames associated with the identified at least one region using the associated IDs of the respective low-resolution frames.

In one embodiment, the method may also include maintaining a queue of the captured plurality of single images for transmitting to the receiver, detecting a queue length exceeding a predetermined maximum queue length, and determining that the bandwidth is below the predetermined threshold.

In one embodiment, the method may also include maintaining a queue of the captured plurality of single images for sending to the receiver, detecting the queue length lower than the predetermined maximum queue length, and determining that the bandwidth is above the predetermined threshold.

Aspects described below include a non-transitory computer-readable storage medium comprising computer-executable instructions that, responsive to execution by a processor, cause a system to perform any one of the described methods.

Aspects described below also include a system with means for generating a high-resolution surface scan of an intraoral cavity.

### BRIEF DESCRIPTION OF THE DRAWINGS

Apparatuses for and techniques for generating a high-resolution surface scan of an intraoral cavity are described with reference to the following drawings. The same numbers are used throughout the drawings to reference like features and components: The drawings are of illustrative embodiments. They do not illustrate all embodiments. Other embodiments may be used in addition or instead. Details that may be apparent or unnecessary may be omitted to save space or for more effective illustration. Some embodiments may be practiced with additional components or steps and/or without all the components or steps that are illustrated. When the same numeral appears in different drawings, it refers to the same or like components or steps.
FIG. 1 depicts a block diagram of a data processing environment in which illustrative embodiments may be implemented.
FIG. 2 depicts a block diagram of a data processing system in which illustrative embodiments may be implemented.
FIG. 3 depicts a schematic diagram of a system in which illustrative embodiments may be implemented.
FIG. 4 depicts a flow chart of a method of generating a high-resolution surface scan, according to an illustrative embodiment of the present disclosure.
FIG. 5 depicts a flow chart of a process of generating a high-resolution surface scan, according to an illustrative embodiment of the present disclosure.
FIG. 6 depicts a flow chart of a process of replacing the low-resolution frames with the residual frames or stitching together, according to an illustrative embodiment of the present disclosure.

### DETAILED DESCRIPTION

### Overview

In the following detailed description, numerous specific details are set forth by way of examples to provide a thorough understanding of the relevant teachings. However, it should be apparent that the present teachings may be practiced without such details. In other instances, well-known methods, procedures, and/or components have been described at a relatively high level, without detail, to avoid unnecessarily obscuring aspects of the present teachings.

The illustrative embodiments recognize that in the field of intraoral imaging, the utilization of intraoral scanners may be integral for obtaining digital impressions of the intraoral cavity. These scanners may play a key role in transmitting recorded data, often utilizing various connectivity options like wireless networks or the public internet. However, a notable challenge may arise when the transmission bandwidth intermittently falls below the rate at which data is being acquired, causing disruptions in smooth data transfer.

The illustrative embodiments recognize that one may attempt to transmit a continuous data stream, and upon experiencing intermittent drops in transmission bandwidth one may drop frames to adjust to the available bandwidth. However, dropping frames may lead to an interruption in the scan flow generating a deteriorated or incomplete surface scan with missing sampled data of certain regions of the intraoral cavity.

The illustrative embodiments implement a method and a system of generating a high-resolution surface scan of the intraoral cavity. As used herein "high-resolution" may generally refer to a resolution that exceeds a predetermined threshold resolution, and "low-resolution" may generally refer to a resolution lower than the predetermined threshold resolution.

In example aspects, a method of generating a high-resolution surface scan of an intraoral cavity is disclosed comprising sampling a surface of the intraoral cavity by capturing a plurality of single images of the surface with an intraoral scanner, detecting a bandwidth of a transmission between the intraoral scanner and a remote receiver, and reducing a resolution of one or more single images responsive to detecting that the bandwidth is lower than a predetermined threshold. The single images may be single "2D images" or single "3D depth images". The resolution of the one or more single images may be reduced by converting the one or more single images into a corresponding/respective one or more low-resolution frames and a corresponding/respective one or more residual frames. The one or more low-resolution frames may be transmitted one after the other to the remote receiver, and sequentially stitched to a model being generated by the receiver, at a first time period wherein the bandwidth is low. Transmitting and stitching the low-resolution frames when the bandwidth is low (below a predetermined threshold) may allow one to continue the intraoral surface scan to generate a stitched model without experiencing interruptions otherwise caused by low bandwidth transmission. At a second time period that begins after the first time period has begun, the low-resolution frames of the stitched model may be selectively updated (such as replaced or combined) with corresponding residual frames within the stitched model to obtain the original high-resolution data of the intraoral scanner and generate a high-resolution overall 3D image of the intraoral cavity. In an aspect, the high-resolution overall 3D image can achieve the same quality and accuracy as if all the high-resolution frames from the camera had been transmitted at the full bandwidth. Further, the selective update may be based on predetermined thresholds of required sampling density such that a predetermined quality can be achieved.

The illustrative embodiments are described with respect to certain types of machines. The illustrative embodiments are also described with respect to other scenes, subjects, measurements, devices, data processing systems, environments, components, and applications only as examples. Any specific manifestations of these and other similar artifacts are not intended to be limiting to the disclosure. Any suitable manifestation of these and other similar artifacts can be selected within the scope of the illustrative embodiments.

Furthermore, the illustrative embodiments may be implemented with respect to any type of data, data source, or access to a data source over a data network. Any type of data storage device may provide the data to an embodiment of the disclosure, either locally at a data processing system or over a data network, within the scope of the disclosure. Where an embodiment is described using a mobile device, any type of data storage device suitable for use with the mobile device may provide the data to such embodiment, either locally at the mobile device or over a data network, within the scope of the illustrative embodiments.

The illustrative embodiments are described using specific code, hardware, algorithms, designs, architectures, protocols, layouts, schematics, and tools only as examples and are not limiting to the illustrative embodiments. Furthermore, the illustrative embodiments are described in some instances using particular software, tools, and data processing environments only as an example for the clarity of the description. The illustrative embodiments may be used in conjunction with other comparable or similarly purposed structures, systems, applications, or architectures. For example, other comparable devices, structures, systems, applications, or architectures therefor, may be used in conjunction with such embodiment of the disclosure within the scope of the disclosure. An illustrative embodiment may be implemented in hardware, software, or a combination thereof.

The examples in this disclosure are used only for the clarity of the description and are not limiting to the illustrative embodiments. Additional data, operations, actions, tasks, activities, and manipulations will be conceivable from this disclosure and the same are contemplated within the scope of the illustrative embodiments.

Any advantages listed herein are only examples and are not intended to be limiting to the illustrative embodiments. Additional or different advantages may be realized by specific illustrative embodiments. Furthermore, a particular illustrative embodiment may have some, all, or none of the advantages listed above.

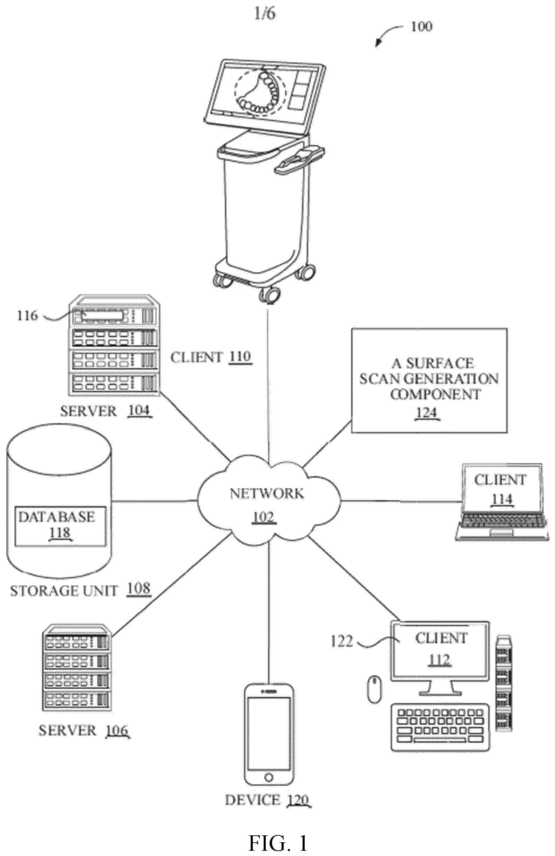

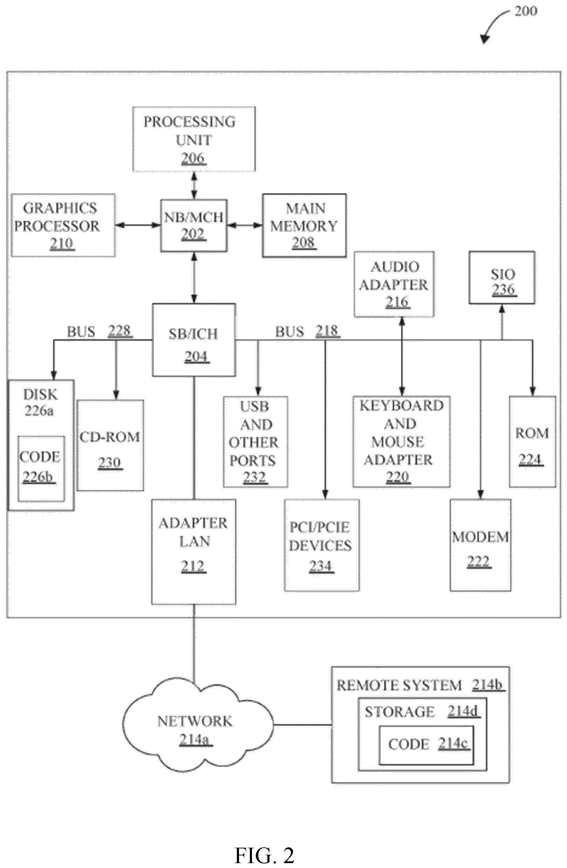

With reference to the figures and in particular, with reference to FIG. 1 and FIG. 2, these figures are example diagrams of data processing environments in which illustrative embodiments may be implemented. FIG. 1 and FIG. 2 are only examples and are not intended to assert or imply any limitation with regard to the environments in which different embodiments may be implemented. A particular implementation may make many modifications to the depicted environments based on the following description.

FIG. 1 depicts a block diagram of a network of data processing systems in which illustrative embodiments may be implemented. Data processing environment 100 is a network of computers in which the illustrative embodiments may be implemented. Data processing environment 100 includes network 102. Network 102 is the medium used to provide communications links between various devices and computers connected together within the data processing environment 100. Network 102 may include connections, such as wire, wireless communication links, or fiber optic cables.

Clients or servers are only example roles of certain data processing systems connected to network 102 and are not intended to exclude other configurations or roles for these data processing systems. Server 104 and server 106 are coupled to network 102 along with storage unit 108. Software applications may execute on any computer in data processing environment 100. Client 110, client 112, and client 114 are also coupled to network 102. A data processing system, such as server 104 or server 106, or clients (client 110, client 112, client 114) may contain data and may have software applications or software tools executing thereon. Server 104 may include one or more GPUs (graphics processing units) for training one or more models.

Only as an example, and without implying any limitation to such architecture, FIG. 1 depicts certain components that are usable in an example implementation of an embodiment. For example, servers and clients are only examples and not to imply a limitation to a client-server architecture. As another example, an embodiment can be distributed across several data processing systems and a data network as shown, whereas another embodiment can be implemented on a single data processing system within the scope of the illustrative embodiments. Data processing systems (server 104, server 106, client 110, client 112, client 114) also represent example nodes in a cluster, partitions, and other configurations suitable for implementing an embodiment.

Device 120 is an example of a device described herein. Any software application described as executing in another data processing system in FIG. 1 can be configured to execute in any of the device herein in a similar manner. Any data or information stored or produced in another data processing system in FIG. 1 can be configured to be stored or produced in any device herein in a similar manner.

A surface scan generation component 124 may execute as part of client application 122, server application 116, or on any data processing system herein. The surface scan generation component 124 may also execute as a cloud service communicatively coupled to system services, hardware resources, or software elements described herein. Database 118 of storage unit 108 stores one or more data in repositories for computations herein. Surface scan generation component 124 may perform a method comprising: sampling a surface of the intraoral cavity by capturing a plurality of single images of the surface with a scanner, detecting a bandwidth of a transmission, reducing a resolution of one or more single images of the plurality of single images associated with at least one region of the surface on detecting the bandwidth to be lower than a predetermined threshold, by converting the one or more single images into a respective low-resolution frames and residual frames associated with the at least one region, and transmitting the respective low-resolution frames to a receiver at the bandwidth. The surface scan generation component 124 may perform stitching, at a first time period, of the respective low-resolution frames to an ongoing model to continue the surface scan to generate a stitched model, selectively replace, at a second time period, the respective low-resolution frames with corresponding residual frames within the stitched model and generate the high-resolution surface scan of the intraoral cavity.

Server application 116 implements an embodiment described herein. Server application 116 can use data from storage unit 108 for generating a high-resolution surface scan of an intraoral cavity. Server application 116 can also obtain data from any client for computations. Server application 116 can also execute in any of data processing systems (server 104 or server 106, client 110, client 112, client 114), such as client application 122 in client 112 and need not execute in the same system as server 104.

Server 104, server 106, storage unit 108, client 110, client 112, client 114, and device 120 may couple to network 102 using wired connections, wireless communication protocols, or other suitable data connectivity. Client 110, client 112, and client 114 may be, for example, personal computers or network computers.

In the depicted example, server 104 may provide data, such as boot files, operating system images, and applications to client 110, client 112, and client 114. Client 110, client 112, and client 114 may be clients to server 104 in this example. Client 110, client 112, and client 114 or some combination thereof, may include their own data, boot files, operating system images, and applications. Data processing environment 100 may include additional servers, clients, and other devices that are not shown. Server 104 includes a server application 116 that may be configured to implement one or more of the functions described herein in accordance with one or more embodiments.

The data processing environment 100 may also be the Internet. Network 102 may represent a collection of networks and gateways that use the Transmission Control Protocol/Internet Protocol (TCP/IP) and other protocols to communicate with one another. At the heart of the Internet is a backbone of data communication links between major nodes or host computers, including thousands of commercial, governmental, educational, and other computer systems that route data and messages. Of course, data processing environment 100 also may be implemented as a number of different types of networks, such as for example, an intranet, a local area network (LAN), or a wide area network (WAN). FIG. 1 is intended as an example, and not as an architectural limitation for the different illustrative embodiments.

Among other uses, data processing environment 100 may be used for implementing a client-server environment in which the illustrative embodiments may be implemented. A client-server environment enables software applications and data to be distributed across a network such that an application functions by using the interactivity between a client data processing system and a server data processing system. Data processing environment 100 may also employ a service-oriented architecture where interoperable software components distributed across a network may be packaged together as coherent business applications. Data processing environment 100 may also take the form of a cloud and employ a cloud computing model of service delivery for enabling convenient, on-demand network access to a shared pool of configurable computing resources (e.g., networks, network bandwidth, servers, processing, memory, storage, applications, virtual machines, and services) that can be rapidly provisioned and released with minimal management effort or interaction with a provider of the service.

With reference to FIG. 2, this figure depicts a block diagram of a data processing system in which illustrative embodiments may be implemented. Data processing system 200 is an example of a computer, such as server 104, server 106, or client (scanner unit/acquisition unit) 110, client 112, client 114, the surface scan generation component 124 in FIG. 1, or another type of device in which computer-usable program code or instructions implementing the processes may be located for the illustrative embodiments.

Data processing system 200 is also representative of a data processing system or a configuration therein, such as device 120 in FIG. 1 in which computer-usable program code or instructions implementing the processes of the illustrative embodiments may be located. Data processing system 200 is described as a computer only as an example, without being limited thereto. Implementations in the form of other devices, such as device 120 in FIG. 1, may modify data processing system 200, such as by adding a touch interface, and even eliminate certain depicted components from data processing system 200 without departing from the general description of the operations and functions of data processing system 200 described herein.

In the depicted example, data processing system 200 employs a hub architecture including North Bridge and memory controller hub (NB/MCH) 202 and South Bridge and input/output (I/O) controller hub (SB/ICH) 204. Processing unit 206, main memory 208, and graphics processor 210 are coupled to North Bridge and memory controller hub (NB/MCH) 202. Processing unit 206 may contain one or more processors and may be implemented using one or more heterogeneous processor systems. Processing unit 206 may be a multi-core processor. Graphics processor 210 may be coupled to North Bridge and memory controller hub (NB/MCH) 202 through an accelerated graphics port (AGP) in certain implementations.

In the depicted example, local area network (LAN) adapter 212 is coupled to South Bridge and input/output (I/O) controller hub (SB/ICH) 204. Audio adapter 216, keyboard and mouse adapter 220, modem 222, read only memory (ROM) 224, universal serial bus (USB) and other ports 232, and PCI/PCIe devices 234 are coupled to South Bridge and input/output (I/O) controller hub (SB/ICH) 204 through bus 218. Hard disk drive (HDD) or solid-state drive (SSD) 226a and CD-ROM 230 are coupled to South Bridge and input/output (I/O) controller hub (SB/ICH) 204 through bus 228. PCI/PCIe devices 234 may include, for example, Ethernet adapters, add-in cards, and PC cards for notebook computers. PCI uses a card bus controller, while PCIe does not. Read only memory (ROM) 224 may be, for example, a flash binary input/output system (BIOS). Hard disk drive (HDD) or solid-state drive (SSD) 226a and CD-ROM 230 may use, for example, an integrated drive electronics (IDE), serial advanced technology attachment (SATA) interface, or variants such as external-SATA (eSATA) and micro- SATA (mSATA). A super I/O (SIO) device 236 may be coupled to South Bridge and input/output (I/O) controller hub (SB/ICH) 204 through bus 218.

Memories, such as main memory 208, read-only memory (ROM) 224, or flash memory (not shown), are some examples of computer usable storage devices. Hard disk drive (HDD) or solid-state drive (SSD) 226a, CD-ROM 230, and other similarly usable devices are some examples of computer usable storage devices including a computer usable storage medium.

An operating system runs on processing unit 206. The operating system coordinates and provides control of various components within data processing system 200 in FIG. 2. The operating system may be a commercially available operating system for any type of computing platform, including but not limited to server systems, personal computers, and mobile devices. An object-oriented or other type of programming system may operate in conjunction with the operating system and provide calls to the operating system from programs or applications executing on data processing system 200.

Instructions for the operating system, the object-oriented programming system, and applications or programs, such as server application 116 and client application 122 in FIG. 1, are located on storage devices, such as in the form of codes 226b on Hard disk drive (HDD) or solid-state drive (SSD) 226a, and may be loaded into at least one of one or more memories, such as main memory 208, for execution by processing unit 206. The processes of the illustrative embodiments may be performed by processing unit 206 using computer-implemented instructions, which may be located in a memory, such as, for example, main memory 208, read-only memory (ROM) 224, or in one or more peripheral devices.

Furthermore, in one case, code 226b may be downloaded over network 214a from remote system 214b, where similar code 214c is stored on a storage device 214d in another case, code 226b may be downloaded over network 214a to remote system 214b, where downloaded code 214c is stored on a storage device 214d.

The hardware in FIG. 1 and FIG. 2 may vary depending on the implementation. Other internal hardware or peripheral devices, such as flash memory, equivalent non-volatile memory, or optical disk drives and the like, may be used in addition to or in place of the hardware depicted in FIG. 1 and FIG. 2. In addition, the processes of the illustrative embodiments may be applied to a multiprocessor data processing system.

In some illustrative examples, data processing system 200 may be a personal digital assistant (PDA), which is generally configured with flash memory to provide non-volatile memory for storing operating system files and/or user-generated data. A bus system may comprise one or more buses, such as a system bus, an I/O bus, and a PCI bus. Of course, the bus system may be implemented using any type of communications fabric or architecture that provides for a transfer of data between different components or devices attached to the fabric or architecture.

A communications unit may include one or more devices used to transmit and receive data, such as a modem or a network adapter. A memory may be, for example, main memory 208 or a cache, such as the cache found in North Bridge and memory controller hub (NB/MCH) 202. A processing unit may include one or more processors or CPUs.

The depicted examples in FIG. 1 and FIG. 2 and above-described examples are not meant to imply architectural limitations. For example, data processing system 200 also may be a tablet computer, laptop computer, or telephone device in addition to taking the form of a mobile or wearable device.

Where a computer or data processing system is described as a virtual machine, a virtual device, or a virtual component, the virtual machine, virtual device, or the virtual component operates in the manner of data processing system 200 using virtualized manifestation of some or all components depicted in data processing system 200. For example, in a virtual machine, virtual device, or virtual component, processing unit 206 is manifested as a virtualized instance of all or some number of hardware processing units 206 available in a host data processing system, main memory 208 is manifested as a virtualized instance of all or some portion of main memory 208 that may be available in the host data processing system, and Hard disk drive (HDD) or solid-state drive (SSD) 226a is manifested as a virtualized instance of all or some portion of Hard disk drive (HDD) or solid-state drive (SSD) 226a that may be available in the host data processing system. The host data processing system in such cases is represented by data processing system 200.

### Method and System for generating a high-resolution surface scan of an intraoral cavity

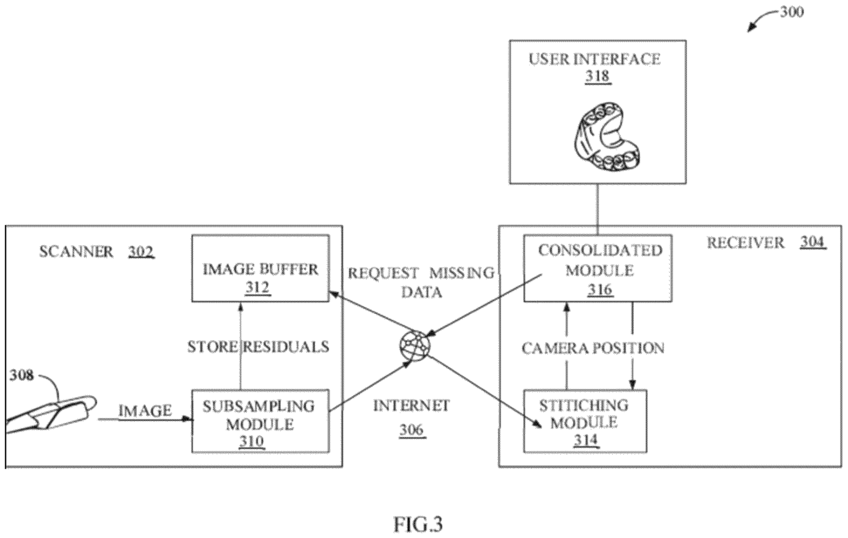

FIG. 3 depicts a schematic diagram of a system in which illustrative embodiments may be implemented. System 300 is an example of a combination of a client 110 (scanner unit or acquisition unit), surface scan generation component 124, and a computer, such as a server 104 and server 106 for scanning an intraoral cavity of a patient and generation of a high-resolution surface scan of the intraoral cavity. The system 300 includes a scanner unit 302. The scanner unit 302 may be a portable device used to sample the surface of the intraoral cavity by capturing a plurality of high-resolution single images of the surface. The scanner unit 302 may include or be formed by a dental camera 308 used to take single images of the intraoral cavity. The scanner unit 302 may be connected to a receiver 304 through a network 306. The receiver 304 is a remote computing device or a cloud computing device that receives the plurality of single images from the scanner unit 302 through the network 306. The scanner unit transmits the single images of the intraoral cavity continuously to the receiver 304 using the network 306 when the network 306 is detected of a sufficient or high bandwidth. The sufficient or high bandwidth can be a bandwidth of the network 306 exceeding a predetermined threshold of bandwidth. The receiver 304 stitches the received single images from the scanner 302 unit to generate a high-resolution consolidated surface scan of the intraoral cavity.

With reference to FIG. 3, the scanner unit 302 further comprises a subsampling module 310 and an image buffer 312. Subsampling module 310, without a limitation, may be a computer algorithm stored within the dental camera 308. The subsampling module 310 is used to subsample the high-resolution single images when the bandwidth of the transmission of the network 306 is detected to be low. The low bandwidth can be a bandwidth of the network 306 that is lower than the predetermined threshold. The subsampling includes decreasing the high-resolution single images into a low-resolution frames and residual frames. The residual frames may be the same as the high-resolution single images or may alternatively be a remainder of the high-resolution images after the subsampling. Further, the subsampling module 310, on detection of a low bandwidth of the transmission, may transmit the low-resolution frames to continue scanning of the surface, while storing the residual frames within the image buffer 312 of the scanner unit 302. Image buffer 312 can be, without any restriction and limitation, a storage module of the dental camera 308 that stores the residual frames of the respective low-resolution frames for the receiver 304 to request and receive later. The residual frame may in some cases be a higher resolution frame that are generated after removing a low-resolution frame from a high-resolution single image. Therefore, in such a case, the single image can be regenerated by stitching the respective low-resolution frame and the residual frame together with an appropriate stitching algorithm.

On detection of the low bandwidth of the transmission, instead of dropping the high-resolution images and interrupting scanning of the surface, the scanner unit 302 using the subsampling module 310 decreases the resolution of the single image to convert the single image into low-resolution frame. Further, at the low bandwidth, the scanner transmits the respective low-resolution frame of the single image to the receiver via the network 306. This decrease in resolution may enable the uninterrupted transmission of the frames to the receiver even on the low bandwidth.

Receiver 304 is used to stitch the received plurality of high-resolution single images and the low-resolution frames in a sequence of their reception from the scanner unit 302. The receiver 304 includes a stitching module 314 that is used to stitch the sequentially received single images and the low-resolution frames together to generate a stitched model. A consolidation module 316 is included within the receiver 304 data communicatively coupled with the stitching module 314. The consolidation module 316 is used to continuously monitor the stitched model generated by the stitching module 314 and request one or more residual frames from the image buffer 312 of the respective one or more low-resolution frames stitched within the stitched model responsive to detecting that the bandwidth is sufficient or high relative to a predetermined threshold. The consolidated module 316 can be connected with the image buffer 312 through the network 306 to send a request and receive the one or more residual frames of the respective low-resolution frames stitched within the stitched model on high bandwidth of the network. The stitching module 314 stitches the received residual frames with their respective low-resolution frames within the stitched module (or replaces the low-resolution frames with the residual frames in cases where the residual frames are of sufficient quality or of same quality as the original single images of the dental camera 308) to generate a high-resolution consolidated model of the scanned surface or intraoral cavity.

Receiver 304, with reference to FIG. 3, can be a server such as server 104, server 106, a remote computing device, or a cloud server comprising the surface scan generation component 124. The stitching module 314 and the consolidated module 316 can be a computer program codes stored within a storage of the receiver 304. In one embodiment, the receiver 304 is a remote computer device comprising a processing unit having a stitching module 314 and the consolidated module 316 that can sequentially receive the single images and the low-resolution frames from the scanner based on the bandwidth of the network 306 and stitch the single images and the low-resolution frames together to generate the stitched model.

In one embodiment, the subsampling module 310 generates and associates IDs with each low-resolution frames and the respective residual frames. The residual frames with their associated IDs may further be stored within the image buffer 312 to enable the consolidated module 316 to request the residual frame using the associated ID of that residual frame. The consolidated module 316 monitors the stitched model generated by the stitching module 314 and identifies at least one region of a surface within the stitched model having a low sampling rate. The low sampling rate is a sampling rate that is lower than a predetermined sampling rate. Further, the consolidated module 316 requests the image buffer to send the residual frames of the respective low-resolution frames present within the said identified at least one region with low sampling rate by using the IDs associated with the residual frames.

In another embodiment, the consolidated module 316 determines a position of the dental camera 308 using the low-resolution frames and requests the image buffer 312 to send the high-resolution residual frames or single images associated with the same position of the dental camera 308 to generate a high-resolution consolidated model of the intraoral cavity.

Further, in an embodiment, the stitching module 314 stitches the received residual frames with their respective low-resolution frames to generate the high-resolution consolidated model. In another embodiment, the stitching module 314 replaces the low-resolution frame with the corresponding residual frame received from the image buffer 312 to generate the high-resolution consolidated model of the intraoral cavity.

The receiver 304 can present the high-resolution consolidated model or surface scan of the intraoral cavity within a user interface 318.

The scanner unit 302 transmits the single images on high bandwidth and the low-resolution frames on low bandwidth of the transmission in a sequence of their capture or generation. The scanner maintains a queue of the captured single images in a sequence to be transmitted to the receiver. Further, the scanner unit 302 detects if the queue length exceeds or is lower than a predetermined maximum queue length. If the queue length is lower than the predetermined maximum queue length, the scanner determines that the bandwidth of the transmission is high, and transfers the high-resolution single images captured by the dental camera 308. On the contrary, if the queue length exceeds the predetermined maximum queue length, the scanner determines that the bandwidth of the transmission is low and converts the single images into the low-resolution frames and residual frames. To detect the bandwidth, the receiver sends acknowledgement signals to the sender. This leads to taking a respective image out of the queue and being able to deduce a low bandwidth from the queue length. Further, the scanner unit 302 transmits the low-resolution frames on the low bandwidth of transmission to the receiver and while storing the residual frames within the image buffer 312 to be requested by the consolidated module 316 of the receiver 304 later responsive to detecting a high bandwidth of the transmission.

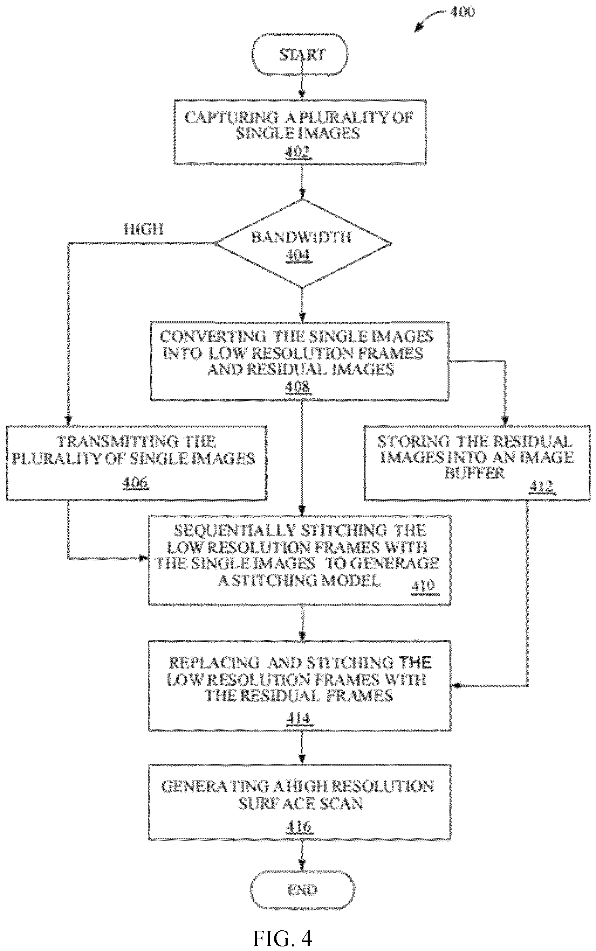

FIG. 4 depicts a flow diagram of a process 400 of generating a high-resolution surface scan, according to an illustrative embodiment of the present disclosure. In block 402, a plurality of single images is captured. The dental camera 308 is provided and the plurality of single images associated with each region of the surface of the intraoral cavity are continuously captured for transmission to the receiver 304 to generate a high-resolution scanned model of the intraoral cavity. In block 404, a bandwidth of the transmission of the network connecting the scanner unit 302 and the receiver 304 is determined. In block 406, the plurality of single images is transmitted to the receiver 304 from the scanner unit 302 when it is computed that the bandwidth of the transmission is high. Conversely, in block 408, the single images are converted into low-resolution frames and transmitted to the receiver 304 when it is determined that the bandwidth is low. In block 410, the low-resolution frames are stitched sequentially with the single images to generate a stitched model. In block 412, the corresponding residual frames of the low-resolution frames are stored within an image buffer to receive later when it is detected that the bandwidth of transmission is high. In block 414, the receiver 304 receives the residual frames and replaces the stitched low-resolution frames with the respective residual frames. Successively, in block 416, the receiver 304 generates a high-resolution consolidated model which is a high-resolution surface scan model of the intraoral cavity.

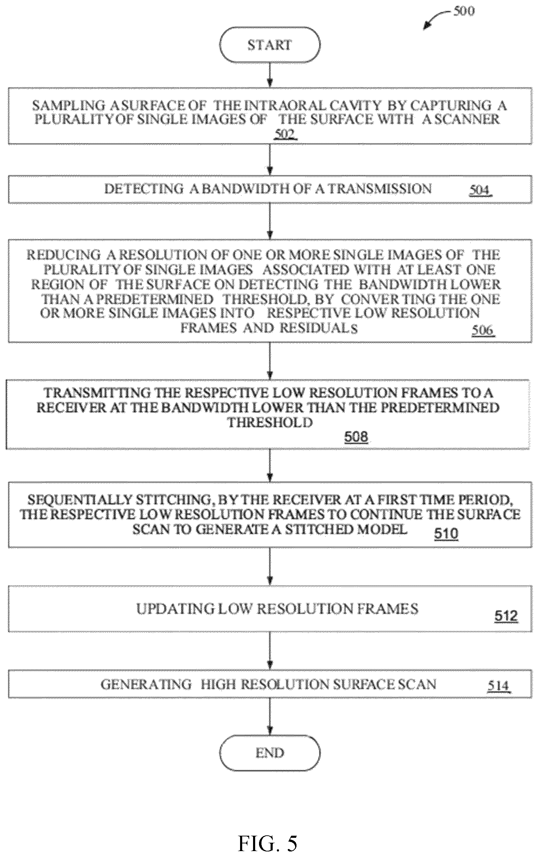

FIG. 5 depicts a flowchart of a process 500 of generating a high-resolution surface scan, according to an illustrative embodiment of the present disclosure. The process 500 is an example illustration of the similar process 400. In block 502, the intraoral cavity is scanned by capturing a plurality of single images of the surface of the intraoral cavity/teeth with a dental camera 308. In block 504, the bandwidth of transmission is detected by comparing a queue of transmission with the predetermined maximum queue length. In block 506, the resolution of the single images is reduced by converting the single images into a respective low-resolution frames and residual frames when there is a low bandwidth of transmission. In block 508, the scanner unit 302 transmits the low-resolution frames to the receiver 304 during the low bandwidth of transmission. In block 510, the receiver 304 sequentially stitches, at a first time period, the low-resolution frames with the single images to continue the surface scanning and generate a stitched model. In block 512, the low-resolution frames are selectively updated based on the corresponding residual frames within the stitched model by requesting and receiving the residual frames from the scanner unit 302. In block 514, the high-resolution surface scan or consolidated model is generated for the intraoral cavity.

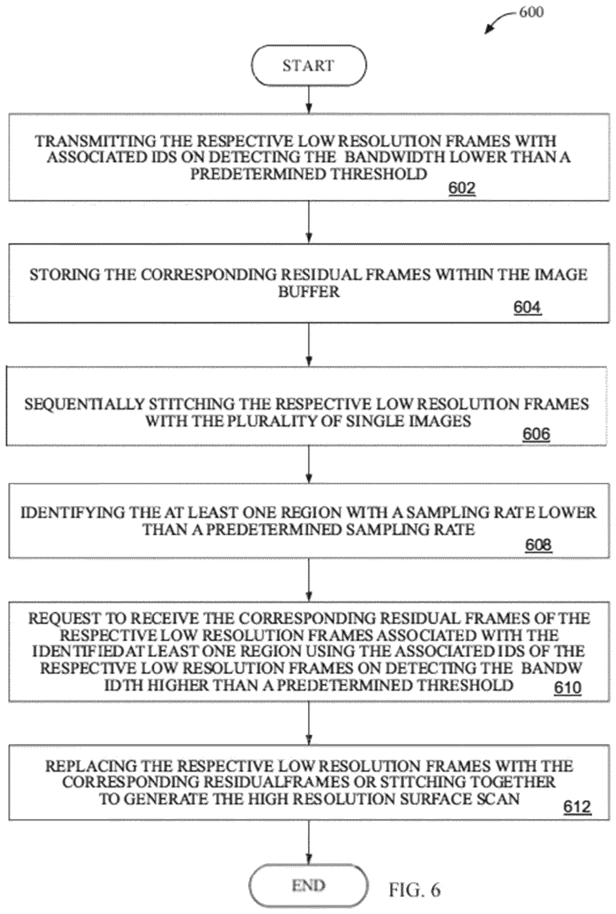

FIG. 6 depicts a flow chart of a process 600 of replacing the low-resolution frames with the residual frames or stitching together, according to an illustrative embodiment of the present disclosure. Process 600 is an example illustration of blocks 508, 510, and 512 of the process 500. In block 602, the scanner unit 302 transmits the low-resolution frames with associated IDs when the bandwidth is detected to be lower than the predetermined threshold. In block 604, the corresponding residual frames with the associated IDs are stored within the image buffer 312. In block 606, the received low-resolution frames are sequentially stitched with a plurality of existing single images or already generated model of other parts of the oral cavity to generate an updated model. In block 608, the consolidation module 316 identifies the at least one region in the model with a sampling rate lower than a predetermined sampling rate within the stitched model. In block 610, the consolidation module 316 requests to receive the corresponding residual frames of the respective low-resolution frames associated with the identified at least one region using the associated IDs of the respective low-resolution frames on detecting the bandwidth to be higher than a predetermined threshold. In block 612, the consolidation module 316 replaces the respective low-resolution frames with the corresponding residual frames or stitches them together to generate a high-resolution surface scan.

### Conclusion

Any specific manifestations of these and other similar example processes are not intended to be limiting to the disclosure. Any suitable manifestation of these and other similar example processes can be selected within the scope of the illustrative embodiments.

Thus, a computer-implemented method, system or apparatus, and computer program product are provided in the illustrative embodiments for generating a high-resolution surface scan of an intraoral cavity and other related features, functions, or operations. Where an embodiment or a portion thereof is described with respect to a type of device, the computer-implemented method, system or apparatus, the computer program product, or a portion thereof, are adapted or configured for use with a suitable and comparable manifestation of that type of device.

Where an embodiment is described as implemented in an application, the delivery of the application in a Software as a Service (SaaS) model is contemplated within the scope of the illustrative embodiments. In a SaaS model, the capability of the application implementing an embodiment is provided to a user by executing the application in a cloud infrastructure. The user can access the application using a variety of client devices through a thin client interface such as a web browser, or other light-weight client-applications. The user does not manage or control the underlying cloud infrastructure including the network, servers, operating systems, or the storage of the cloud infrastructure. In some cases, the user may not even manage or control the capabilities of the SaaS application. In some other cases, the SaaS implementation of the application may permit a possible exception of limited user-specific application configuration settings.

The present disclosure may be a system, a method, and/or a computer program product at any possible technical detail level of integration. The computer program product may include a computer-readable storage medium (or media) having computer-readable program instructions thereon for causing a processor to carry out aspects of the present disclosure.

The computer-readable storage medium can be a tangible device that can retain and store instructions for use by an instruction execution device. The computer-readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer-readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer-readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer-readable program instructions described herein can be downloaded to respective computing/processing devices from a computer-readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer-readable program instructions from the network and forwards the computer-readable program instructions for storage in a computer-readable storage medium within the respective computing/processing device.

Computer-readable program instructions for carrying out operations of the present disclosure may be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, including an object oriented programming language such as Smalltalk, C++, or the like, and procedural programming languages, such as the "C" programming language or similar programming languages. The computer-readable program instructions may execute entirely on a dedicated system or user's computer, partly on the user's computer or dedicated system as a stand-alone software package, partly on the user's computer and partly on a remote computer or entirely on the remote computer or server, etc. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer-readable program instructions by utilizing state information of the computer-readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to embodiments of the disclosure. It will be understood that each block of the flowchart illustrations and/or block diagrams, and combinations of blocks in the flowchart illustrations and/or block diagrams, can be implemented by computer-readable program instructions.

These computer-readable program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer-readable program instructions may also be stored in a computer-readable storage medium that can direct a computer, a programmable data processing apparatus, and/or other devices to function in a particular manner, such that the computer-readable storage medium having instructions stored therein comprises an article of manufacture including instructions which implement aspects of the function/act specified in the flowchart and/or block diagram block or blocks.

The computer-readable program instructions may also be loaded onto a computer, other programmable data processing apparatus, or other device to cause a series of operational steps to be performed on the computer, other programmable apparatus or other device to produce a computer-implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present disclosure. In this regard, each block in the flowchart or block diagrams may represent a module, segment, or portion of instructions, which comprises one or more executable instructions for implementing the specified logical function(s). In some alternative implementations, the functions noted in the blocks may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

All features disclosed in the specification, including the claims, abstract, and drawings, and all the steps in any method or process disclosed, may be combined in any combination, except combinations where at least some of such features and/or steps are mutually exclusive. Each feature disclosed in the specification, including the claims, abstract, and drawings, can be replaced by alternative features serving the same, equivalent, or similar purpose, unless expressly stated otherwise.

## Claims

1. A method of generating a high-resolution surface scan of an intraoral cavity, the method comprising:
sampling a surface of the intraoral cavity by capturing a plurality of single images of the surface with a scanner;
computing a bandwidth of a transmission;
reducing a resolution of one or more single images of the plurality of single images associated with at least one region of the surface responsive to computing that the bandwidth is lower than a predetermined threshold, by converting the one or more single images into respective one or more low-resolution frames and respective one or more residual frames associated with the at least one region; and
sequentially transmitting the respective one or more low-resolution frames to a receiver at the bandwidth;
sequentially stitching, by the receiver at a first time period, the respective one or more low-resolution frames to continue the surface scan to generate a stitched model;
selectively updating, at a second time period that starts after the first time period starts, the respective one or more low-resolution frames based the respective one or more residual frames within the stitched model; and
generating the high-resolution surface scan of the intraoral cavity.

2. The method of claim 1, wherein:
the respective one or more residual frames have a resolution that is the same as or different from the resolution of the one or more single images,
responsive to the respective one or more residual frames having a resolution that is lower than the resolution of the one or more single images, the respective one or more low-resolution frames are updated by adding the respective one or more residual frames to the respective one or more low-resolution frames, and
responsive to the respective one or more residual frames having a resolution that is the same as the resolution of the one or more single images, the respective one or more low-resolution frames are updated by replacing the respective one or more low-resolution frames with the respective one or more residual frames.

3. The method of claim 1 or claim 2, wherein sequentially stitching the respective one or more low-resolution frames comprises registering and stitching the respective one or more low-resolution frames associated with the at least one region in a sequence with the plurality of the single images of remaining regions already scanned to generate the stitched model of the surface.

4. The method of any of claims 1 to 3, wherein the scanner stores the respective one or more residual frames of the one or more single images within an image buffer of the scanner.

5. The method of claim 4, wherein the receiver requests to receive the respective one or more residual frames from the scanner on detecting that the bandwidth is above the predetermined threshold.

6. The method of claim 5, wherein the respective one or more low-resolution frames are transmitted with associated identifications (IDs) to enable requesting of the respective one or more residual frames from the scanner by the receiver.

7. The method of claim 6, wherein:
the receiver maps a first surface of the stitched model to identify a first region with a sampling rate lower than a predetermined sampling rate; and
the receiver requests for the first region the respective one or more residual frames of the respective one or more low-resolution frames associated with the first region using the associated IDs of the respective one or more low-resolution frames;
optionally wherein the received respective one or more residual frames replaces the respective one or more low-resolution frames or are stitched together to generate the high-resolution surface scan.

8. The method of any of claims 1 to 7, wherein detecting the bandwidth comprises:
maintaining a queue of the captured plurality of single images for transmitting to the receiver;
detecting a queue length exceeding a predetermined maximum queue length based on acknowledgement signals from the receiver; and
determining that the bandwidth is below the predetermined threshold;
optionally wherein detecting the bandwidth further comprises:
maintaining a queue of the captured plurality of single images for sending to the receiver;
detecting the queue length lower than the predetermined maximum queue length; and
determining that the bandwidth is above the predetermined threshold;
further optionally wherein the scanner transmits the plurality of single images of the surface on detecting the bandwidth that is above the predetermined threshold; and
further optionally wherein the plurality of the single images are high-resolution images of the surface.

9. The method of any of claims 7 to 8, further comprising:
identifying a second surface of the stitched model with the sampling rate higher than the predetermined sampling rate; and
reducing the resolution of the one or more single images associated with second surface.

10. The method of any of claims 7 to 9, further comprising:
visualizing, over a user interface, at least one region within the stitched model with the sampling rate lower than the predetermined sampling rate; and
guiding a user to capture one or more new single images from said at least one region using the scanner.

11. A system for generating high-resolution surface scan of an intraoral cavity, the system comprising:
at least one processor configured to,
sample a surface of the intraoral cavity by capturing a plurality of single images of the surface with a scanner;
compute a bandwidth of a transmission;
reduce a resolution of one or more single images of the plurality of single images associated with at least one region of the surface responsive to computing that the bandwidth is lower than a predetermined threshold, by converting the one or more single images into respective one or more low-resolution frames and respective one or more residual frames associated with the at least one region; and
sequentially transmit the respective one or more low-resolution frames to a receiver at the bandwidth;
sequentially stitch, by the receiver at a first time period, the respective one or more low-resolution frames to continue the surface scan to generate a stitched model;
selectively update, at a second time period subsequent to the first time period, the respective one or more low-resolution frames with the respective one or more residual frames within the stitched model; and
generate the high-resolution surface scan of the intraoral cavity.

12. The system of claim 11, wherein the receiver is configured to register and stitch the respective one or more low-resolution frames associated with the at least one region in a sequence with the plurality of the single images of other regions of the intraoral cavity to generate the stitched model.

13. The system of claim 12, wherein the receiver is configured to,
receive the respective one or more residual frames of the respective low-resolution frames from the scanner responsive to detecting that the bandwidth is higher than the predetermined threshold;
selectively replace the respective one or more low-resolution frames with the respective one or more residual frames within the stitched model; and
generate the high-resolution surface scan of the intraoral cavity;
optionally wherein the scanner comprises an image buffer configured to store the respective one or more residual frames of the one or more single images.

14. The system of any of claims 11 to 13, further comprising:
a user interface configured to,
visualize at least one region within the stitched model with a sampling rate lower than a predetermined sampling rate; and
guide a user to capture one or more new single images from said at least one region using the scanner.

15. A non-transitory computer readable storage medium storing one or more programs that when executed by a computer cause the computer to:
sample a surface of the intraoral cavity by capturing a plurality of single images of the surface with a scanner;
compute a bandwidth of a transmission;
reduce a resolution of one or more single images of the plurality of single images associated with at least one region of the surface responsive to computing that the bandwidth is lower than a predetermined threshold, by converting the one or more single images into respective one or more low-resolution frames and respective one or more residual frames associated with the at least one region; and
sequentially transmit the respective one or more low-resolution frames to a receiver at the bandwidth;
sequentially stitch, by the receiver at a first time period, the respective one or more low-resolution frames to continue the surface scan to generate a stitched model;
selectively update, at a second time period that starts after the first time period starts, the respective one or more low-resolution frames with the respective one or more residual frames within the stitched model; and
generate the high-resolution surface scan of the intraoral cavity.
